# EUROPEAN PATENT SPECIFICATION

(11) **EP 1 255 811 B1**
(45) Date of publication and mention of the grant of the patent: **30.06.2010**
(21) Application number: 01915260.2
(22) Date of filing: 15.02.2001
(51) Int. Cl.: C12N 1/20, C12P 17/16, C12P 17/18

(54) **SINGLE COLONIES OF MYXOBACTERIA CELLS**
EINZELZELLKOLONIEN VON MYXOBAKTERIEN
COLONIES UNIQUES DE CELLULES MYXOBACTERIENNES

(30) Priority: 17.02.2000 GB 0003753
(43) Date of publication of application: 13.11.2002
(73) Proprietor: Novartis AG, 4056 Basel (CH); Novartis Pharma GmbH, 1230 Wien (AT)
(72) Inventor: STROHHÄCKER, Joachim, A-6323 Bad Häring (AT)
(74) Representative: Gros, Florent
(86) International application number: PCT/EP2001/001686
(87) International publication number: WO 2001/060976

(56) References cited:
- WO-A-93/10121
- US-A- 5 686 295
- KARWOWSKI J P ET AL: "A method for the selective isolation of Myxococcus directly from soil." JOURNAL OF INDUSTRIAL MICROBIOLOGY, vol. 16, no. 4, 1996, pages 230-236, XP001010286 ISSN: 0169-4146
- BRETSCHER A P ET AL: "NUTRITION OF MYXOCOCCUS-XANTHUS A FRUITING MYXOBACTERIUM" JOURNAL OF BACTERIOLOGY, vol. 133, no. 2, 1978, pages 763-768, XP001010255 EN ISSN: 0021-9193

## Description

The present invention relates to myxobacteria microorganisms.

Myxobacteria microorganisms are widely spread soil bacteria including e.g. myxobacterium *Sorangium,* such as myxobacterium *Sorangium cellulosum*. Similar to Streptomycetes, myxobacteria bacteria may produce secondary metabolites in a high structural diversity, e.g. epothilones, such as epothilone A and epothilone B. Myxobacteria bacteria have an outstandig characterestic: they are able to glide.

Bacterium cells may generally form single colonies, e.g. which may originate from a single cell, if cultivated on the surface of an, e.g. semi-solid, nutrient medium, e.g. which contains agar agar. No single colonies, however, but agglomerates of bacterium cells originating from the whole surface of the nutrient medium may be obtained, if Myxobacteria cells are cultivated because myxobacteria cells may be able to move, e.g. to glide or to swarm on a surface of an, e.g. semi-solid, nutrient medium, e.g. which contains agar agar. Thus, cultivation of single colonies of myxobacteria cells, e.g. originating from a single cell, may be difficult, e.g. practically impossible if a myxobacteria cell suspension is cultivated on an, e.g. semi-solid, nutrient medium.

It was now found that single colonies of myxobacteria cells surprisingly may be obtained on an, e.g. semi-solid, nutrient medium despite of the ability of myxobacteria cells to move, e.g. to glide or to swarm on a surface of an, e.g. semi-solid, nutrient medium.

In one aspect the present invention provides a single colony of myxobacteria cells.

A single colony as described herein includes e.g. a cell colony originating from one single colony-forming unit of myxobacteria cells, e.g. in the form of a distinct colony with defined borders, e.g. which may be visibly recognised as a single colony. The term "single colony" as defined herein means a colony which originates from a small number, e.g. less than four, of myxobacteria cells, preferably, from a single myxobacteria cell.

Myxobacteria include preferably myxobacterium *Sorangium*, more preferably myxobacterium *Sorangium cellulosum*. Myxobacteria cells include preferably cells of myxobacterium *Sorangium*, more preferably cells of myxobacterium *Sarangium cellulosum.*

In another aspect the present invention provides a process for production of single colonies of myxobacteria *Sorangium* comprising cultivating myxobacteria *Sorangium* on a nutritient medium containing 1 g/l to 5 g/l isoleucin and/or leucin, e.g. isoleucin; or isoleucin and leucin.

A process according to the present invention may be carried out as follows:
An appropriate nutrient medium includes a nutrient medium on which myxobacteria cells can grow. Preferably the nutrient medium is semi-solid, e.g. the nutrient medium contains agar agar. Nutrient medium for the growth of myxobacteria is known as such and production thereof may e.g. be carried out according to a method as conventional.
Typically a nutrient medium for the growth of myxobacteria cells may contain
   - an assimilable carbon source, e.g glucose;
   - an assimilable nitrogen source, e.g NH₄⁺, e.g. in the form of (NH₄)₂SO₄, a pancreatin digest of casein, such as bacto tryptone, e.g. commercially available from Difco;
   - a phosphor source, e.g a phosphate, such as KH₂PO₄;
   - trace elements, e.g. Mg, e.g. in the form of MgSO₄, Ca, e.g. in the form of CaCl₂, Fe, such as Fe-EDTA (EDTA: ethylenediamine tetraacetic acid);
   - optionally a sulphur source, e.g a sulphate, such as Na₂SO₄, (NH₄)₂SO₄;
optionally buffer compounds, e.g. (3-N-morpholino)propanesulfonic acid (MOPS).
The nutrient medium may contain further and other appropriate ingredients.

According to the present invention the nutrient medium contains isoleucin and/or leucin. Preferably the nutrient medium contains isoleucin, or isoleucin and leucin. Isoleucin and leucin include L-isoleucin and L-leucin.

It was found that isoleucin and/or leucin present in the nutrient medium may inhibit the gliding or swarming of myxobacteria cells an the surface of the nutrient medium.

Isoleucin and/or leucin are present in the nutrient medium In an amount sufficient for inhibiting the gliding, e.g. swarming of myxobacteria cells on the nutrient medium. An appropriate amount of isoleucin and/or leucin in the nutrient medium includes e.g. 0.5 g to 10 g isoleucin and/or leucin per litre of nutrient medium, such as 0.8 g/l to 6 g/l, e.g. 1 g/l to 5 g/l.

The nutrient medium containing isoleucin and/or leucin, e.g. in semi-solid form, e.g. containing agar agar, may be placed on a support, e.g. a plate or a dish, to obtain a support with an, e.g. semi-solid, nutrient medium containing isoleucin and/or leucin on the surface.

A support with an, e.g. semi-solid, nutrient medium containing isoleucin and/or leucin on the surface may be inoculated with a myxobacteria cell suspension.

Myxobacteria cells may be otained from myxobacteria, including e.g. myxobacterium *Sorangium,* such as myxobacterium *Sorangium cellulosum* microorganisms which are well known, e.g. commercially available, e.g. according to a method as conventional.

An appropriate myxobacteria cell suspension, e.g. a liquid culture, of myxobacteria cells may be obtained by incubating a liquid culture medium with myxobacteria cells. A liquid culture medium for the incubation of myxobacteria cells includes a culture medium on which myxobacteria cells can grow. An appropriate liquid culture medium is known or may be prepared, e.g., according to a method as conventional. A typical liquid culture medium for incubating myxobacteria cells may, e.g., contain sources for assimilable carbon, assimilable nitrogen etc, such as glucose, starch, soya (flour), yeast extract; and trace elements, e.g. Mg, e.g. in the form of MgSO₄, Ca, e.g. in the form of CaCl₂, Fe, e.g. In the form of Fe-EDTA.

Incubation may be carried out for an appropriate time, e.g. several days, at an appropriate temperature, e.g. 20°C to 40°C, e.g. around 30°C, e.g. under shaking. Myxobacteria cells obtained may be centrifugated off and resuspended in fresh liquid culture medium. The suspension obtained may be diluted using an appropriate dilution medium, such as an enzymatic hydrolysate of soyabean meal, e.g. in diluted form, e.g. in 1% to 10% solution, such as 5% solution, e.g. diluted with water, such as a solution of Bacto Soytone, e.g. commercially available from Difco. A cell suspension with a dilution rate of up to 10⁻⁶ may e.g. be appropriate.

A diluted suspension of myxobacteria cells which is appropriate for inoculation of nutrient medium, e.g. on a support, may be obtained.

An inoculated support with an, e.g. semi-solid, nutrient medium containing isoleucin and/or leucin on the surface may be incubated, e.g. according to a method as conventional, e.g.
- at appropriate temperatures, such as 30°C to 40°C, e.g. 37°C;
- for an appropriate time, e.g. for several days, e.g. 10 to 20 days, such as 12 to 14 days.

Single colonies of myxobacteria cells on the surface of the nutrient medium may be obtained, e.g. having a diameter of several mm, such as 4 to 5 mm after an appropriate time. The single colonies may be distinct and may be visibly recognised as single colonies, e.g. having defined borders.

Cell density may be determined as usual, e.g. in a counting chamber, e.g. in a Thoma-chamber.

Single colonies of myxobacteria cells are useful for effective strain improvement.

Myxobacteria cells are able to produce secondary metabolites, which e.g. may be usful as a pharmaceutical. For example, myxobacterium *Sorangium* is able to produce the known pharmaceutically active epothilones, especially epothilone A and epothilone B, but also epothilone D, having the following formulae, 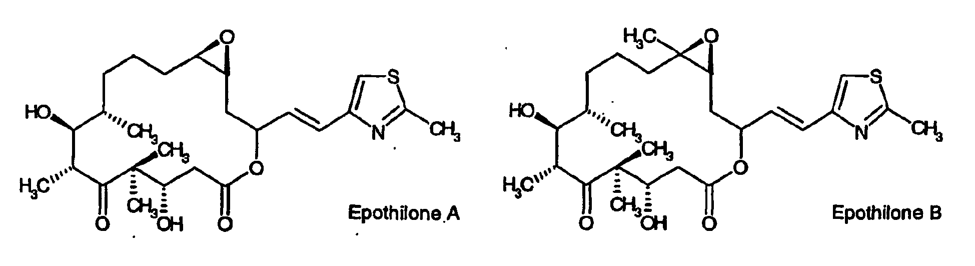 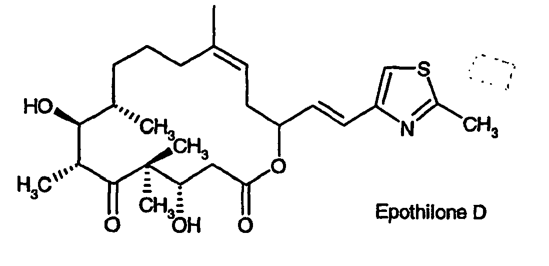 which are known to inhibit the proliferation of tumor cells and are suitable for the treatmnet of tumor diseases (see, e.g., Bolag, D.M. et al., "Epothilones, a new class of microtubule-stabilizing agents with a Taxol-like mechanism of action", Cancer Research 55, 2325-33 (1995), Kowalski, R.J. et al., J. Biol. Chem. 272(4), 2534-2541 (1997), US 5,641,803, US 5,496,804, US 5,565,478; for epothilone A see especially WO93/10121 and for epothilone D especially WO 99/01124).

Strain improvement of myxobacteria strains, e.g. myxobacterium *Sorangium* may e.g. improve the production rate of secondary metabolites, e.g. pharmaceutically active compounds, e.g. epothilones.

If cells undergo a pre-treatment, e.g. mutation or cell transformation by corresponding treatment, and such cells can only be obtained in the form of aggregated cells and not in the form of single colonies, cells of different genotypes may be aggregated. In such case a cell selection of cells having different, especially desired, characteristics is impossible, because cells with different characteristics after treatment cannot be selected, if single colonies cannot be formed. With other words, the formation of single colonies is a necessary or at least very advantageous precondition for carrying out a process of selection of cells having desired characteristics.

If single colonies can be formed, which is enabled according to the present invention for myxobacteria, e.g. myxobacterium *Sorangium* cells, effective selection of cells having different characteristics after a corresponding pre-treatment may be carried out, e.g. of cells having a higher production rate of a secondary metabolite, e.g. a desired, e.g. pharmaceutically active, compound, e.g. epothilones, than untreated cells.

In another aspect the present invention provides the use of a single colony of myxobacteria cells in the improvement of myxobacteria strains, e.g. myxobacterium *Sorangium* strains; and, in another aspect the production of a myxobacterium *Sorangium* strain having an improved epothilone production rate, comprising the steps:
i) producing single colonies of pre-treated myxobacterium *Sorangium* cells having an improved epothilone production rate compared with untreated myxobacterium *Sorangium* cells on a nutrient medium which includes 1 g/l to 5 g/l isoleucin and/or leucin,
ii) selecting cells from the single colonies obtained under step i) having an improved epothilone production rate compared with untreated myxobacterium *Sorangium* cells;
iii) cultivating cells selected in step ii) having an improved epothilone production rate compared with untreated myxobacterium *Sorangium* cells.

Step i) may e.g. be carried out according to the present invention. Steps ii) and iii) may be carried out e.g. according to a conventional method.

Cell pre-treatment which may improve the production rate of the epothilones compared with untreated myxobacterium *Sorangium* cells include e.g. transformation of myxobacterium *Sorangium* cells, cell treatment which results in mutagenesis of myxobacterium *Sorangium* cells, spontaneous mutagenesis. Such pre-treatment is, e.g., described in US 5,686,295.

The present invention relates in particular to a myxobacterium *Sorangium* strain having ar improved epothilone production rate obtained by the process described above.

Furthermore, the present invention relates to the use of a nutritient medium containing isoleucine and/or leucine in a process for the Improvement of myxobacteria strains.

In another aspect the present invention provides a process for the production of epothilones, e.g. epothilone B, comprising the steps
i) forming single colonies of pre-treated myxobacterium *Sorangium* cells having an improved epothilone production rate compared with untreated myxobacterium *Sorangium* cells on a nutrient medium which includes 1 g/l to 5 g/l isoleucin and/or leucin,
ii) selecting cells from the single colonies obtained under step i) having an improved epothilone production rate compared with untreated myxobacterium *Sorangium* cells;
iii) cultivating cells selected in step ii) having an improved epothilone production rate compared with untreated myxobacterium *Sorangium* cells;
iv) fermenting cultivated myxobacterium *Sorangium* cells obtained in step iii); and
v) isolating epothilone from the fermentation broth.

In another aspect the present invention provides a myxobacterium *Sorangium* strain having an improved epothilone production rate.

Step I) may e.g. be carried out according to the present invention. Steps ii) to v) may be carried out e.g. according to a conventional method.

In the folllowing examples all temperatures are uncorrected and given in °Celsius.

### Example 1

### a. Semi-solid nutrient medium for plating

| Solution A: 700 ml | |
|---|---|
| Bacto Tryptone | 0,71 g/l |
| MgSO₄ . 7 H₂O | 2,1 g/l |
| (NH₄)₂SO₄ | 0,71 g/l |
| CaCl₂ . 2H₂O | 1,4 g/l |
| MOPS | 17 g/l |
| ((3-N-morpholinol)propanesulfonic acid) | |
| Solution B | 1,4 ml/l |
| Agar agar | 28 g/l |
| L-isoleucin | 1 g/l |
| L-leucin | 2 g/l |

Water in an amount to obtain 700 ml of Solution A.
The pH of solution A is adjusted with NaOH to 7.4.

| Solution B: | |
|---|---|
| Fe-EDTA in water | 8 g/l |
| | |
| Solution C: 100 ml | |
| Glucose in water | 35 g/l |
| | |
| Solution D: 100 ml | |
| KH₂PO₄ in water | 0,6 g/l |
| | |
| Solution E: 10 ml | |
| Na₂S₂O₄ in water | 10 g/l |
| (0,2 µm filter-sterilized) | |

### Solution F: 35 ml

Commercially available *Sorangium cellulosum* microorganism (cells) in liquid culture form, treated in autoclave, 3 days old.

Solutions A, C, D and F are separately treated in an autoclave and mixed after cooling at around 60°. To the mixture a freshly prepared solution E is added shortly before pouring the nutrient medium onto plates.
The mixture obtained is poured onto plates. Plates with the nutrient medium on the surface are obtained.

### b. Liquid culture medium for cultivating myxobacteria, e.g. myxobycterium Sorangium cellulosum

| Solution A: 50 ml | |
|---|---|
| Glucose | 2 g/l |
| Starch | 8 g/l |
| Soya flour | 2 g/l |
| Yeast extract | 2 g/l |
| MgSO₄ . 7 H₂O | 1 g/l |
| CaCl₂. 2H₂O | 1 g/l |
| Solution B | 1 ml/l |

Water in an amount to obtain 50 ml of solution A.
The pH of solution A is adjusted to 7.4 with NaOH. Sterilisation is carried out for ca. 20 minutes at 122°.

| Solution B: | |
|---|---|
| Fe-EDTA | 8 g/l |

A liquid culture of myxobacterium *Sorangium cellulosum* is incubated in liquid nutrient medium for ca. 3 days at ca. 30°C and 180 Upm. The culture obtained is sterile centrifugated off and resuspended in fresh liquid nutrient medium. The cell suspension obtained is diluted with 0.5% Bacto Soytone solution (aqueous enzymatic hydrolysate of soyabean meal) to obtain dilution series up to a dilution of 10⁻⁶.

### c. Formation of single colonies of myxobacterium Sorangium cellulosum cells

Plates with the nutrient medium on the surface obtained as described under a) are inoculated with dilution series of myxobacterium *Sorangium cellulosum* obtained under b.

Per plate 100 µl of the dilution series are used for inoculation.
The inoculated plates are incubated at ca. 37°C for a period of ca. 12 to 14 days.

### d. Results

Distinct single colonies of myxobacterium *Sorangium cellulosum* cells having defined borders and having a diameter of 4 to 5 mm are obtained. The colonies are recognised visibly as single colonies.

Cell density, determined by counting in a Thoma-chamber is typically 3.7 x 10⁸ cells/ml. At acell suspension dilution rate of 10⁻⁵ 180 single colonies are obtained on a plate, corresponding to a living bacterial count of 1,8 x 10⁸ cells/ml.
Retrieval rate: 49 %.

### Example 2

Example 1 is repeated with the difference that solution A contains 2 g of L-isoleucin and no L-leucin instead of 2 g of L-leucin and 1 g of L-isoleucin.
Similar results as indicated in example 1 are obtained.

## Claims

1. A process for the production of single colonies of myxobacterium *Sorangium* comprising cultivating myxobacterium *Sorangium* on a nutritient medium including 1 g/l to 5g/l of isoleucin and/or leucin.

2. Use of a nutritient medium including 1 g/l to 5g/l of isoleucine and/or leucine in a process for the improvement of myxobacterium *Sorangium* strains.

3. A process for the production of a myxobacterium *Sorangium* strain having an improved epothilone production rate comprising the steps
i) forming single colonies of pre-treated myxobaycterium *Sorangium* cells having an improved epothilone production rate compared with untreated myxobaycterium *Sorangium* cells on a nutrient medium which includes 1 g/l to 5g/l of isoleucin and/or leucin;
ii) selecting cells from the single colonies obtained under step i) having an improved epothilone production rate compared with untreated myxobacterium *Sorangium* cells;
iii) cultivating cells selected in step ii) having an improved epothilone production rate compared with untreated myxobacterium *Sorangium* cells.

4. A process for the production of epothilone comprising the steps
i) forming single colonies of pre-treated myxobaycterium *Sorangium* cells having an improved epothilone production rate compared with untreated myxobaycterium *Sorangium* cells on a nutrient medium which includes between 1 g/l to 5g/l of isoleucin and/or leucin:
ii) selecting cells from the single colonies obtained under step i) having an improved epothilone production rate compared with untreated myxobacterium *Sorangium* cells;
iii) cultivating cells selected in step ii) having an improved epothilone production rate compared with untreated myxobacterium *Sorangium* cells.;
iv) fermenting cultivated cells obtained in step iii); and
v) isolating epothilone from the fermentation broth.

5. A process according to claim 4, wherein epothilone B is produced.

6. A process according to any one of claims 1 or 3 to 5, the use of claim 3 and a strain of claim 6, wherein the myxobacterium is the myxobacterium *Sorangium cellulosum*.

## Patentansprüche

1. Verfahren zur Herstellung einzelner Kolonien von Myxobakterium *Sorangium* durch Züchten von Myxobakterium *Sorangium* auf einem Nährstoffmedium, das 1 g/l bis 5 g/l Isoleucin und/oder Leucin umfasst.

2. Verwendung eines Nährstoffmediums, das 1 g/l bis 5 g/l Isoleucin und/oder Leucin umfasst, in einem Verfahren zur Verbesserung von Myxobakterium *Sorangium*-Stämmen.

3. Verfahren zur Herstellung eines Myxobakterium *Sorangium*-Stamms mit einer verbesserten Epothilonproduktionsrate, das die folgenden Stufen umfasst:
i) Bilden einzelner Kolonien von vorbehandelten Myxobakterium *Sorangium*-Zellen mit einer verbesserten Epothilonproduktionsrate im Vergleich zu nicht behandelten Myxobakterium *Sorangium*-Zellen auf einem Nährstoffmedium, das 1 g/l bis 5 g/l Isoleucin und/oder Leucin umfasst;
ii) Selektieren von Zellen aus den in Stufe i) erhaltenen einzelnen Kolonien mit einer verbesserten Epothilonproduktionsrate im Vergleich zu nicht behandelten Myxobakterium *Sorangium*-Zellen;
iii) Züchten von in Stufe ii) ausgewählten Zellen mit einer verbesserten Epothilonproduktionsrate im Vergleich zu nicht behandelten Myxobakterium *Sorangium-*Zellen.

4. Verfahren zur Herstellung von Epothilon, das die folgenden Stufen umfasst:
i) Bilden von einzelnen Kolonien von vorbehandelten Myxobakterium *Sorangium*-Zellen mit einer verbesserten Epothilonproduktionsrate im Vergleich zu nicht behandelten Myxobakterium *Sorangium*-Zellen auf einem Nährstoffmedium, das zwischen 1 g/l und 5 g/l Isoleucin und/oder Leucin umfasst;
ii) Selektieren von Zellen aus den in Stufe i) erhaltenen einzelnen Kolonien mit einer verbesserten Epothilonproduktionsrate im Vergleich zu nicht behandelten Myxobakterium *Sorangium*-Zellen;
iii) Züchten von in Stufe ii) selektierten Zellen mit einer verbesserten Epothilonproduktionsrate im Vergleich zu nicht behandelten Myxobakterium *Sorangium-*Zellen;
iv) Fermentieren von in Stufe iii) erhaltenen gezüchteten Zellen und
v) Isolieren von Epothilon aus der Fermentationsbrühe.

5. Verfahren nach Anspruch 4, wobei Epothilon B produziert wird.

6. Verfahren nach einem der Ansprüche 1 oder 3 bis 5, Verwendung nach Anspruch 2 und ein Stamm nach Anspruch 6, wobei das Myxobakterium das Myxobakterium *Sorangium cellulosum* ist.

## Revendications

1. Procédé de production de colonies simples de la myxobactérie *Sorangium*, comprenant la mise en culture de la myxobactérie *Sorangium* sur un milieu nutritif comprenant de 1 g/l à 5 g/l d'isoleucine et/ou de leucine.

2. Utilisation d'un milieu nutritif comprenant de 1 g/l à 5 g/l d'isoleucine et/ou de leucine dans un procédé d'amélioration des souches de la myxobactérie *Sorangium*.

3. Procédé de production d'une souche de la myxobactérie *Sorangium* présentant un taux de production d'épothilones amélioré, comprenant les étapes qui consistent à
i) former des colonies simples de cellules prétraitées de la myxobactérie *Sorangium* présentant un taux de production d'épothilones amélioré en comparaison de celui des cellules de la myxobactérie *Sorangium* qui n'ont pas été traitées, sur un milieu nutritif qui comprend de 1 g/l à 5 g/l d'isoleucine et/ou de leucine,
ii) sélectionner, parmi les colonies simples obtenues à l'étape i), les cellules qui présentent un taux de production d'épothilones amélioré en comparaison de celui des cellules de la myxobactérie *Sorangium* qui n'ont pas été traitées ;
iii) mettre en culture les cellules sélectionnées à l'étape ii), à savoir celles qui présentent un taux de production d'épothilones amélioré en comparaison de celui des cellules de la myxobactérie *Sorangium* qui n'ont pas été traitées.

4. Procédé de production d'épothilones comprenant les étapes qui consistent à :
i) former des colonies simples de cellules prétraitées de la myxobactérie *Sorangium* présentant un taux de production d'épothilones amélioré en comparaison de celui des cellules de la myxobactérie *Sorangium* qui n'ont pas été traitées, sur un milieu nutritif qui comprend de 1 g/l à 5 g/l d'isoleucine et/ou de leucine ;
ii) sélectionner, parmi les colonies simples obtenues à l'étape i), les cellules qui présentent un taux de production d'épothilones amélioré en comparaison de celui des cellules de la myxobactérie *Sorangium* qui n'ont pas été traitées ;
iii) mettre en culture les cellules sélectionnées à l'étape ii), à savoir celles qui présentent un taux de production d'épothilones amélioré en comparaison de celui des cellules de la myxobactérie *Sorangium* qui n'ont pas été traitées ;
iv) mettre à fermenter les cellules mises en culture obtenues à l'étape iii) ; et
v) isoler l'épothilone du bouillon de fermentation.

5. Procédé selon la revendication 4, dans lequel on produit de l'épothilone B.

6. Procédé selon l'une quelconque des revendications 1 ou 3 à 5, utilisation selon la revendication 3 et souche selon la revendication 6, dans lesquels la myxobactérie est la myxobactérie *Sorangium cellulosum*.
